# EUROPEAN PATENT SPECIFICATION

(11) **EP 1 663 063 B1**
(45) Date of publication and mention of the grant of the patent: **21.07.2010**
(21) Application number: 04783527.7
(22) Date of filing: 09.09.2004
(51) Int. Cl.: A61F 13/00

(54) **WOUND HEALING APPARATUS WITH BIOABSORBABLE MATERIAL AND SUCTION TUBES**
WUNDHEILUNGSGERÄT MIT BIOABSORBIERBAREM MATERIAL UND SAUGROHREN
APPAREIL DE CICATRISATION DES PLAIES COMPRENANT UNE MATIERE BIOABSORBABLE ET DES TUBES D'ASPIRATION

(30) Priority: 10.09.2003 US 501799 P; 05.04.2004 US 818454
(43) Date of publication of application: 07.06.2006
(73) Proprietor: KCI Licensing, Inc., San Antonio, Texas 78230 (US)
(72) Inventor: WATSON, Richard, L., Jr., McPherson, KS 67640 (US)
(74) Representative: Wardley, Diana Mary
(86) International application number: PCT/US2004/029309
(87) International publication number: WO 2005/025448

(56) References cited:
- WO-A-02/092783
- WO-A-03/028786
- WO-A1-2004/047649
- DE-A1- 4 037 931
- US-A- 6 126 675
- US-A1- 2002 150 604
- US-B1- 6 356 782
- US-B2- 6 855 153

## Description

### BACKGROUND OF THE INVENTION

This invention relates generally to the wound healing arts, and more particularly to a novel wound healing apparatus containing bioabsorbable material for promoting new tissue growth and suction tubes for removing excess fluid from the wound.

### SUMMARY OF THE INVENTION

The present invention is directed to an apparatus as set forth in claim 1 appended hereto.

The apparatus preferably comprises a bioabsorbable mesh fabric made of threads that are absorbable into the human body. Examples of suitable threads for forming the mesh fabric include synthetic absorbable sutures, such as coated VICRYL RAPIDE^{™} (polyglactin 910) sutures available from Ethicon (Somerville, NJ). When placed inside the body, such suture material typically absorbs into the body within about 5 to 10 days. As the mesh fabric is being absorbed, it serves as a framework for fibroblasts to bridge the gap in the wounded tissue and thereby promote healing. The mesh fabric is preferably supported by a skeleton of impervious flexible tubes (such as Teflon^{™} tubes), which are removed from the body after the mesh fabric is absorbed. The flexible tubes also serve as conduits to remove excess fluids from the wound, preferably under the power of a suction device to which the tubes are connected outside the body. The tubes may have fenestrations or openings along their lengths to help remove the excess fluid from the wound. Preferably, an oxygen saturation sensor is incorporated into the apparatus for monitoring the oxygen saturation level of blood in the vicinity of the wound. The present apparatus and method may be used with animals as well as humans.

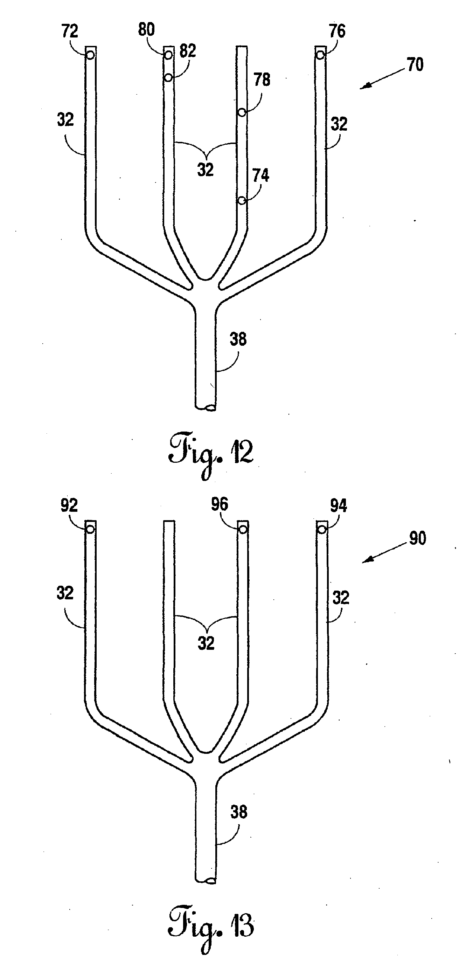

International patent application no. WO03/028786 discloses a wound healing apparatus for treating a wound comprising a plurality of skeletal members and an absorbable fabric supported by said plurality of skeletal members (see, in particular, figures 13 and 14 of the application as published).

### BRIEF DESCRIPTION OF THE DRAWINGS

Fig. 1 is a side view of an expandable embodiment of a wound healing apparatus in accordance with the present invention in an undeployed condition.
Fig. 2 is a side view of the expandable embodiment of Fig. 1 in a deployed condition.
Fig. 3 is a side view of the expandable embodiment of Fig. 1 partially deployed into a wound cavity.
Fig. 4 is a side view of the expandable embodiment of Fig. 1 fully deployed into a wound cavity.
Fig. 5 is a side view of the expandable embodiment of Fig. 1 fully deployed into a wound cavity with the bioabsorbable material having been absorbed.
Fig. 6 is a plan view of an alternative example of a wound healing apparatus in accordance with the present invention.
Fig. 7 is an end view of the alternative example of Fig. 6 taken in the direction of arrows 7-7.
Fig. 8 is an enlarged view of a suction tube.
Fig. 9 is another alternative example.
Fig. 10 is yet another example.
Fig. 11 is still another example.
Fig. 12 is a plan view of yet another example.
Fig. 13 is a plan view of still another alternative example.

### DETAILED DESCRIPTION OF A PREFERRED EMBODIMENT

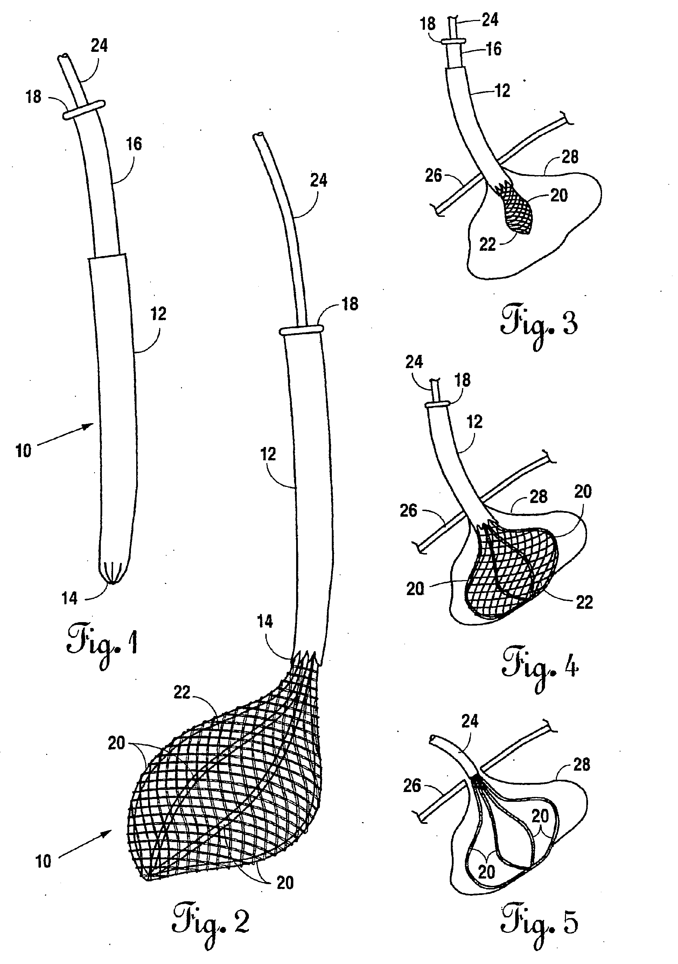

Referring to Figs. 1-5, a wound healing device 10 comprises a bioabsorbable mesh fabric material 22 and flexible tubes 20 arranged to form an expandable bag that is designed to be deployed into a wound cavity 28, such as a seroma, created in human flesh after the removal of a bulk of tissue during surgery or other invasive trauma to the body. Such interior cavities commonly develop after the surgery is completed and the skin 26 is closed. Examples of suitable threads for forming the mesh fabric material 22 include synthetic absorbable sutures, such as coated VICRYL RAPIDE^{™} (polyglactin 910) sutures available from Ethicon (Somerville, NJ). Tubes 20 are preferably made of a material that is impervious to the body, such as Teflon^{™} material. The bag is deployable from an introducer tube 12 that preferably has an expandable tip 14 and a plunger 16. The tip 14 of the introducer tube 12 is inserted through the skin 26 and into the cavity 28 as shown in Fig. 3, and then the plunger 16 is used to deploy and expand the mesh bag within the cavity 28 as shown in Fig. 4. Plunger 16 preferably has a stop 18 to limit the travel of plunger 16 into introducer tube 12. In a preferred embodiment, the deployed bag is shaped similar to a kitchen whisk. After deployment, introducer tube 12 and plunger 16 are removed from the cavity 28. As shown in Fig. 5, approximately 5 to 10 days after deployment, the bioabsorbable mesh fabric 22 will be fully absorbed into the body, leaving only the tubes 20 which may then be removed from the cavity 28. During the healing process, tubes 20 serve as conduits to remove excess fluid from the wound through evacuation tube 24. Preferably, evacuation tube 24 is connected to a suction device such as a vacuum pump (not shown) outside the body to facilitate removal of excess fluid from the cavity 28. A preferred suction device for use with wound healing device 10 is a personally portable vacuum desiccator as described in U.S. Patent No. 6,648,862 issued to the present inventor. Tubes 20 may have fenestrations or openings along their lengths as described for tubes 32 below to help in removal of excess fluid.

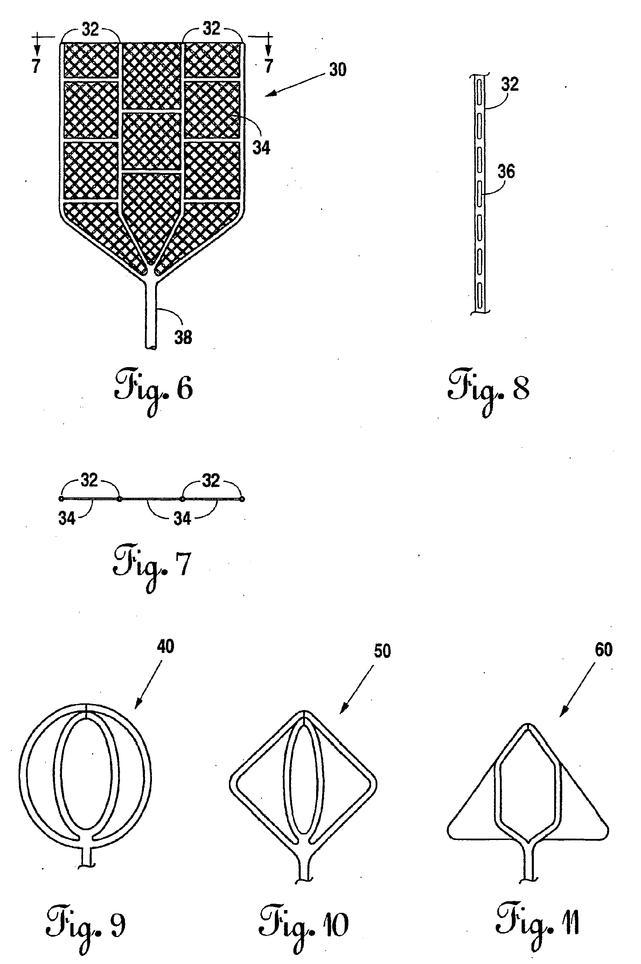

Referring to Figs. 6-8, an alternative wound healing apparatus 30 comprises a bioabsorbable mesh fabric 34 supported by a framework of flexible tubes 32 to form a substantially planar sheet that is designed to be implanted in a surgical wound at the time of surgery. Examples of suitable threads for forming the mesh fabric 34 include synthetic absorbable sutures, such as coated VICRYL RAPIDE^{™} (polyglactin 910) sutures available from Ethicon (Somerville, NJ). Tubes 32 are connected to an evacuation tube 38. Tubes 32 and 38 are preferably made of a material that is impervious to the body, such as Teflon^{™} material. Like the deployable bag embodiment 10 described above, the sheet-like embodiment 30 serves to promote healing through growth of fibroblasts in the wound, and the tubes 32 serve to remove excess fluid from the wound, preferably by connection of evacuation tube 38 to a suction device (not shown), such as a personally portable vacuum desiccator as described in U.S. Patent No. 6,648,862. Tubes 32 preferably have fenestrations or openings 36 to help evacuate excess fluid from the wound. Wound healing apparatus 30 may be placed in the wound toward the end of surgery, and the skin may be closed over apparatus 30 with tube 38 extending out of the wound. Tubes 32 and 38 are removed from the wound after the mesh fabric material 34 is absorbed into the body. The sheet-like example may be made in any of a number of suitable shapes, such as rectangular (see Fig. 6), oval (see Fig. 9), diamond (see Fig. 10), triangular (see Fig. 11), or the like. For the sake of simplicity and clarity, no bioabsorbable fabric material is shown in Figs. 9-11. Such substantially planar example are especially useful in "flap and graft" plastic surgery procedures to help minimize or avoid disfigurement as the wound heals following surgery. Of course, persons of ordinary skill in the art will appreciate that wound healing devices may also be curved, if desired, rather than being substantially flat, depending on the particular wound to be treated.

As an additional benefit, wound healing apparatus 30 may be provided with an oxygen saturation (SaO₂) sensor (not shown) for sensing the oxygen saturation level of the blood in the vicinity of the wound. Referring again to Figs. 6 and 7, an oxygen saturation sensor may be placed in the end of one of the tubes 32, and associated power and signal wires (not shown) may be routed from the sensor through tube 32 and tube 38 to the associated signal analyzer (not shown). Similarly, referring to Figs. 1-5, an oxygen saturation sensor may be included in the end of one of the tubes 20 of wound healing device 10, and the power and signal wires may be routed from the sensor through tubes 20 and 24 to the associated signal analyzer. Suitable oxygen saturation sensors and their operation are well known in the art, one example of which is an OxiMax^{™} sensor available from NellCor Puritan Bennett, Inc. (St. Louis, Missouri). By incorporating an oxygen saturation sensor into a wound healing apparatus in accordance with the present invention, medical personnel are better able to monitor the oxygen saturation level of the blood in the vicinity of the wound as the wound heals in order to evaluate the progress of the healing process.

As persons of ordinary skill in the art will appreciate, other biodegradable materials may be used in lieu of or in addition to the above described bioabsorbable mesh fabric in accordance with the present invention. For example, other suitable biodegradable materials include biodegradable plastics such as beta glucan available from Biopolymer Engineering, Inc. (Eagan, Minnesota), which is an extract from brewer's yeast and also serves as an anti-infectant; polyhydroxyalkanoates (PHAs) available from Degradable Solutions AG (Zurich, Switzerland); and hard gelatins such as those used for ingestible capsules available from Capsugel^{™}, a subsidiary of Pfizer, Inc. (Morris Plains, New Jersey).

Fig. 12 illustrates an alternative wound healing apparatus 70 which is similar to apparatus 30 discussed above except that apparatus 70 also includes an electrocardiogram (ECG) sensor, a carbon dioxide (CO₂) sensor, and an oxygen saturation (SaO₂) sensor, as further described below. Like apparatus 30 described above, apparatus 70 preferably has a bioabsorbable mesh fabric (not shown for the sake of clarity) supported by a framework of flexible tubes 32, which are connected to an evacuation tube 38. Apparatus 70 is used much like apparatus 30 to help heal wounds, but apparatus 70 also provides the capability to monitor ECG activity, CO₂ levels, and SaO₂ levels of blood in the vicinity of the wound during the healing process. To facilitate those monitoring functions, apparatus 70 preferably includes three ECG electrodes 72, 74, 76, a CO₂ sensor 78, and an SaO₂ sensor comprising a light source 80 and a light detector 82 as is known in the art, each of which is preferably disposed on or in one of the tubes 32. Persons of skill in the art will appreciate that the placement of electrodes 72, 74, 76, CO₂ sensor 78, light source 80, and light detector 82 may vary. The associated electrical power and signal lines (not shown) for the ECG sensor, CO₂ sensor, and SaO₂ sensor are preferably routed through tubes 32 and 38 to their respective power sources and signal processors (not shown), the configuration and operation of which are well known in the art. Because apparatus 70 is positionable directly in a wound, the ECG sensor, CO₂ sensor, and SaO₂ sensor of apparatus 70 are capable of providing convenient and accurate information as to the ECG activity, CO₂ levels, and SaO₂ levels of blood in the vicinity of the wound, which greatly assists caregivers in monitoring the progress of the healing process.

A CO₂ sensor 78 as described for apparatus 70 above is particularly useful in monitoring a wound for infection when the bioabsorbable material of apparatus 70 contains a PHA material. As is known in the art, PHA material degrades into CO₂ and water, and the rate of degradation is markedly increased by elevated levels of bacteria. Thus, if a wound containing PHA material is infected, the bacteria will break down the PHA material at a faster rate, which will increase the rate of production of CO₂. Accordingly, CO₂ sensor 78 serves as a convenient means of monitoring the wound for infection. In cooperation with its signal processor, CO₂ sensor 78 preferably provides a visual or audible indication if the CO₂ level in the wound reaches or exceeds a certain predetermined level so that a caregiver may check the wound for infection. Alternatively, if a wound being treated with apparatus 70 is known to be infected, CO₂ sensor 78 and its signal processor may cooperate to provide a visual or audible indication if the CO₂ level in the wound drops below a certain predetermined level so that a caregiver may know that the infection has sufficiently decreased. The CO₂ sensor 78 may be provided either as part of the framework of tubes 32 as shown in Fig. 12, or downstream in the evacuation tube 38, or as part of the vacuum source that is connected to evacuation tube 38. The YSI 8500 CO₂ sensor available from YSI Incorporated (Yellow Springs, Ohio) is an example of a suitable CO₂ sensor that is adaptable for use in accordance with the present invention.

Fig. 13 illustrates another alternative wound healing apparatus 90 which is similar to apparatus 30 discussed above except that apparatus 90 also includes a pair of electrodes 92 and 94 for electric stimulation of the wound and a pressure transducer 96 for measuring blood pressure in the vicinity of the wound. Pressure transducer 96 is preferably a micro pressure transducer such as a Mikro-Tip^{™} SPR series pressure transducer available from Millar Instruments, Inc. (Houston, Texas) or an Accutorr Plus^{™} sensor available from Datascope Corporation (Montvale, New Jersey). Like apparatus 30 described above, apparatus 90 preferably has a bioabsorbable mesh fabric (not shown for the sake of clarity) supported by a framework of flexible tubes 32, which are connected to an evacuation tube 38. Apparatus 90 is used much like apparatus 30 to help heal wounds, but apparatus 90 also provides the capability to stimulate the flesh in the wound with electricity, which further promotes healing as is known in the art, and the capability to monitor the blood pressure in the vicinity of the wound, which serves as an indication of whether the wound is healing properly and the general health condition of the patient. Electrodes 92 and 94 and pressure transducer 96 are preferably disposed on or in one of the tubes 32. Persons of skill in the art will appreciate that the placement of electrodes 92 and 94 and pressure transducer 96 may vary. The associated electrical power lines (not shown) for electrodes 92 and 94 and the associated electrical power lines and signal lines (not shown) for pressure transducer 96 are preferably routed through tubes 32 and 38 to their respective power sources and signal processors (not shown), the configuration and operation of which are well known in the art.

Referring again to Figs. 12 and 13, the aforementioned ECG sensor 72, 74, 76, CO₂ sensor 78, SaO₂ sensor 80, 82, pressure transducer 96, and electric stimulation electrodes 92, 94 may all be provided in the same wound healing apparatus. If an ECG sensor and electric stimulation electrodes are provided in the same apparatus, the ECG sensor and the electric stimulation electrodes are preferably not operated at the same time to avoid electrical interference. Additionally, because the blood being monitored by the SaO₂ sensor 80, 82 is generally pulsing through blood vessels in the flesh at a certain frequency, the signal received by the detector 82 will be periodic, and the period of that signal is indicative of the patient's heart rate. Thus, the heart rate may be calculated from the SaO₂ signal, preferably by a computerized signal processor (not shown). Similarly, the ECG electrodes 72, 74, 76 may be used to measure the difference in bioimpedance of adjacent bodily tissue, such as the chest wall, when the patient is inhaling versus when the patient is exhaling and thereby calculate the patient's respiratory rate. The results of the ECG, CO₂, SaO₂, blood pressure, heart rate, and respiratory rate measurements and calculations may be displayed on a monitor (not shown) according to methods well known in the art.

Persons of ordinary skill in the art will appreciate that tubes 20 and 24 of device 10 shown in Figs. 2-5 and tubes 32 and 38 of devices 30, 70, and 90 shown in Figs. 6, 12, and 13, respectively, may also be used to inject medicine into the wound. For example, liquid antibiotics, angiogenic factors, keratin-based medicine, or other suitable medicines may be injected into the wound to help promote healing. Additionally, medicine may be embedded in the bioabsorbable mesh fabric 22 (see Fig. 2) and 34 (see Fig. 6) or other biodegradable material to help promote healing. For example, a conventional antibiotic such as ciprofloxacin or a lyophilized (freezedried) antibiotic that becomes activated upon contact with moisture may be embedded in the bioabsorbable material to help promote healing. Similarly, a keratin-based substance or an angiogenic substance may be embedded in the bioabsorbable material to help promote healing and stimulate the creation of new blood vessels.

Although tubes 20 (see Figs. 2-5) and 32 (see Fig. 6) described above are preferably made of an impervious material such as Teflon^{™}, tubes 20 and 32 may be made of a biodegradable material that gets absorbed into the body over a period of time as the wound heals. In such an embodiment, eventually nothing would remain to be pulled out of the wound, and tube 24 or 38 would simply break away from the wound site after a period of time. Examples of suitable biodegradable materials for tubes 20 and 32 may include biodegradable plastics, such as beta glucans, PHAs, and hard gelatins such as those used for ingestible capsules, as described above.

Although the foregoing specific details describe a preferred embodiment of this invention, persons reasonably skilled in the art will recognize that various changes may be made.

For example, although the bioabsorbable fabric is generally described herein as a mesh fabric, which is preferably made in a uniform woven fashion, persons of ordinary skill in the art will appreciate that the bioabsorbable fabric may be made in any suitable form, including a nonwoven form, and the openings between the threads forming the fabric and the arrangement of the threads themselves may be non uniform rather than uniform. Also, although the framework for supporting the bioabsorbable material is preferably comprised of a plurality of flexible tubes in order to provide the capability to remove excess fluid from the wound and to inject medicine into the wound through the tubes, the framework may be comprised of one or more solid, elongated rods, if desired, and such rods may be used in conjunction with or in lieu of tubes. Accordingly, as used herein, the term "skeletal member" means any flexible member suitable for carrying a bioabsorbable fabric or other biodegradable material in accordance with this invention, which may or may not have a conduit, such as a tube, for removing fluid from the wound. Although the skeletal members support the bioabsorbable fabric or other biodegradable material, the bioabsorbable fabric or other biodegradable material may or may not be attached to the skeletal members, such as by adhesive or heat sealing. For example, the fibers of the bioabsorbable fabric may be looped around the skeletal members. As another example, although the flexible framework and bioabsorbable material of one preferred embodiment are deployable as an expandable bag, the flexible framework and bioabsorbable material need not necessarily form an expendable bag; some other suitable deployed form may be desirable. Persons of ordinary skill in the art will also appreciate that any of the sensors or electrical stimulation electrodes described herein may be used with any wound healing apparatus described herein. Additionally, many other variations of the present invention are possible. Therefore, it should be understood that this invention is not to be limited to the specific details shown and described herein.

## Claims

1. A wound healing apparatus (10) for treating a wound, comprising:
a plurality of skeletal members (20); and
a bioabsorbable fabric (22) supported by said plurality of skeletal members (20)
**characterised in that**
said plurality of skeletal members (20) and said bioabsorbable fabric (22) form an expandable bag that is deployable from an insertion tube (12) with a plunger (16).

2. The wound healing apparatus (10) claim 1 wherein at least one of said plurality of skeletal members (20) comprises a conduit for removing fluid from the wound.

3. The wound healing apparatus (10) of claim 2 wherein said conduit is placeable in fluid communication with a suction device to assist in removing fluid from the wound.

4. The wound healing apparatus (10) of claim 2 or claim 3 wherein said conduit is adaptable for injecting medicine into the wound.

5. The wound healing apparatus (10) of any preceding claim further comprising at least one sensor (72, 74, 76, 78, 80) connected to at least one of said plurality of skeletal members (20); wherein said at least one sensor (72, 74, 76, 78, 80) is selected from the group consisting of an oxygen saturation sensor, a carbon dioxide sensor, an electrocardiogram sensor, and a blood pressure sensor.

6. The wound healing apparatus (10) of claim 5 wherein:
said at least one sensor comprises a carbon dioxide sensor (78); and
said bioabsorbable fabric (22) comprises a PHA material.

7. The wound healing apparatus (10) of any preceding claim further comprising a plurality of electrodes (72, 74, 76, 78, 80) connected to at least one of said plurality of skeletal members;
said plurality of electrodes (72, 74, 76, 78, 80) being adaptable for providing electrical stimulation to the wound.

8. The wound healing apparatus (10) of any preceding claim wherein said plurality of skeletal members (20) comprises a biodegradable material.

9. A wound healing apparatus (10) according to claim 1 wherein:
said apparatus further comprises an evacuation tube;
each of the said plurality of skeletal members (20) comprises a flexible tube connected to said evacuation tube; and
wherein said bioabsorbable fabric (22) is supported by said flexible tubes (20);
wherein said flexible tubes (20) are adaptable for removing fluid from the wound through said evacuation tube; and
wherein said flexible tubes (20) are removable from the wound after said bioabsorbable fabric (22) is absorbed by the wound.

10. The wound apparatus (10) of claim 9 comprising
an insertion tube (12) in which said flexible tubes (20) and said bioabsorbable fabric (22) are initially disposed; and
a plunger (16) operably connected to said plurality of flexible tubes (20);
wherein said insertion tube (12) is insertable into a wound; and
wherein said plunger (16) is operable for deploying said flexible tubes (20) and said bioabsorbable fabric (22) from said insertion tube (12) into the wound;

11. The wound healing apparatus (10) of any one of claims 9 to 10 wherein said evacuation tube is adaptable for connection to a suction device to assist in removing fluid from the wound.

12. The wound healing apparatus (10) of any one of claims 9 to 11 further comprising at least one sensor connected to at least one of said flexible tubes (20);
wherein said at least one sensor is selected from the group consisting of an oxygen saturation sensor, a carbon dioxide sensor, an electrocardiogram sensor, and a blood pressure sensor.

13. The wound healing apparatus (10) of claim 12 wherein:
said at least one sensor comprises a carbon dioxide sensor; and
said bioabsorbable fabric comprises a PHA material.

14. The wound healing apparatus (10) of any one of claims 9 to 13 wherein said evacuation tube and at least one of said flexible tubes (20) are adaptable for injecting medicine into the wound.

15. The wound healing apparatus (10) of any one of claims 9 to 14 wherein said bioabsorbable fabric comprises medicine embedded therein.

16. The wound healing apparatus (10) of any one of claims 9 to 15 wherein said plurality of flexible tubes (20) comprise a biodegradable material.

17. The wound healing apparatus (10) of any one of claims 9 to 16 further comprising a plurality of electrodes connected to at least one of said flexible tubes;
said plurality of electrodes being adaptable for providing electrical stimulation to the wound.

18. The wound apparatus (10) of any preceding claim wherein said apparatus is adaptable for placement in a wound.

## Patentansprüche

1. Wundheilungsgerät (10) zum Behandeln einer Wunde, umfassend: mehrere Skelettelemente (20); und ein bioabsorbierbares Gewebe (22), das von den mehreren Skelettelementen (20) gehalten wird, **dadurch gekennzeichnet, dass** die mehreren Skelettelemente (20) und das bioabsorbierbare Gewebe (22) einen dehnbaren Beutel bilden, der mit einem Stößel (16) aus einem Einführungsröhrchen (12) entfaltet werden kann.

2. Wundheilungsgerät (10) nach Anspruch 1, wobei zumindest eines der mehreren Skelettelemente (20) eine Leitung zum Entfernen von Flüssigkeit aus der Wunde umfasst.

3. Wundheilungsgerät (10) nach Anspruch 2, wobei die Leitung zur Unterstützung beim Entfernen von Flüssigkeit aus der Wunde in Fluidverbindung mit einer Absaugvorrichtung platziert werden kann.

4. Wundheilungsgerät (10) nach Anspruch 2 oder Anspruch 3, wobei die Leitung dazu ausgelegt werden kann, Arznei in die Wunde zu injizieren.

5. Wundheilungsgerät (10) nach irgendeinem vorhergehenden Anspruch, weiterhin mit zumindest einem Sensor (72, 74, 76, 78, 80), der mit zumindest einem der mehreren Skelettelemente (20) verbunden ist; wobei der zumindest eine Sensor (72, 74, 76, 78, 80) ausgewählt ist aus der Gruppe, bestehend aus einem Sauerstoffsättigungssensor, einem Kohlendioxidsensor, einem Elektrokardiogrammsensor und einem Blutdrucksensor.

6. Wundheilungsgerät (10) nach Anspruch 5, wobei: der zumindest eine Sensor einen Kohlendioxidsensor (78) umfasst; und das bioabsorbierbare Gewebe (22) ein PHA-Material umfasst.

7. Wundheilungsgerät (10) nach irgendeinem vorhergehenden Anspruch, weiterhin mit mehreren Elektroden (72, 74, 76, 78, 80), die mit zumindest einem der mehreren Skelettelemente verbunden sind; wobei die mehreren Elektroden (72, 74, 76, 78, 80) dazu ausgelegt werden können, der Wunde eine elektrische Stimulation zuzuführen.

8. Wundheilungsgerät (10) nach irgendeinem vorhergehenden Anspruch, wobei die mehreren Skelettelemente (20) ein biologisch abbaubares Material umfassen.

9. Wundheilungsgerät (10) nach Anspruch 1, wobei: das Gerät weiterhin ein Entleerungsröhrchen umfasst; jedes der mehreren Skelettelemente (20) ein elastisches Röhrchen umfasst, das mit dem Entleerungsröhrchen verbunden ist; und wobei das bioabsorbierbare Gewebe (22) von den elastischen Röhrchen (20) gehalten ist; wobei die elastischen Röhrchen (20) dazu ausgelegt werden können, Flüssigkeit aus der Wunde durch den Entleerungsröhrchen zu entfernen; und wobei die elastischen Röhrchen (20) aus der Wunde entfernt werden können, nachdem das bioabsorbierbare Gewebe (22) von der Wunde absorbiert ist.

10. Wundheilungsgerät (10) nach Anspruch 9, umfassend ein Einführungsröhrchen (12), in dem die elastischen Röhrchen (20) und das bioabsorbierbare Gewebe (22) anfangs angeordnet sind; und einen Stößel (16), der funktionsfähig mit den mehreren elastischen Röhrchen (20) verbunden ist; wobei das Einführungsröhrchen (12) in eine Wunde eingeführt werden kann; und wobei der Stößel (16) dazu funktionsfähig ist, die elastischen Röhrchen (20) und das bioabsorbierbare Gewebe (22) aus dem Einführungsröhrchen (12) in die Wunde zu entfalten.

11. Wundheilungsgerät (10) nach irgendeinem der Ansprüche 9 bis 10, wobei das Einführungsröhrchen für eine Verbindung mit einer Absaugvorrichtung zur Unterstützung bei dem Entfernen von Flüssigkeit aus der Wunde ausgelegt werden kann.

12. Wundheilungsgerät (10) nach irgendeinem der Ansprüche 9 bis 11, weiterhin mit zumindest einem Sensor, der mit zumindest einem der elastischen Röhrchen (20) verbunden ist; wobei der zumindest eine Sensor ausgewählt ist aus der Gruppe, bestehend aus einem Sauerstoffsättigungssensor, einem Kohlendioxidsensor, einem Elektrokardiogrammsensor und einem Blutdrucksensor.

13. Wundheilungsgerät (10) nach Anspruch 12, wobei: der zumindest eine Sensor einen Kohlendioxidsensor umfasst; und das bioabsorbierbare Gewebe ein PHA-Material umfasst.

14. Wundheilungsgerät (10) nach irgendeinem der Ansprüche 9 bis 13, wobei das Entleerungsröhrchen und zumindest eines der elastischen Röhrchen (20) dazu ausgelegt werden können, Arznei in die Wunde zu injizieren.

15. Wundheilungsgerät (10) nach irgendeinem der Ansprüche 9 bis 14, wobei das bioabsorbierbare Gewebe Arznei umfasst, die in dasselbe eingebettet ist.

16. Wundheilungsgerät (10) nach irgendeinem der Ansprüche 9 bis 15, wobei die mehreren elastischen Röhrchen (20) ein biologisch abbaubares Material umfassen.

17. Wundheilungsgerät (10) nach irgendeinem der Ansprüche 9 bis 16, weiterhin mit mehreren Elektroden, die mit zumindest einem der elastischen Röhrchen verbunden sind; wobei die mehreren Elektroden dazu ausgelegt werden können, der Wunde eine elektrische Stimulation zuzuführen.

18. Wundheilungsgerät (10) nach irgendeinem vorhergehenden Anspruch, wobei das Gerät für eine Platzierung in einer Wunde ausgelegt werden kann.

## Revendications

1. Un appareil de cicatrisation des blessures (10) pour soigner une blessure, composé de plusieurs membres d'ossature (20) et d'un tissu bioabsorbable (22) soutenu par lesdits membres d'ossature (20), **se caractérisant par le fait que** les membres d'ossature (20) et le tissu bioabsorbable (22) forment un sac expansible déployable à partir d'un tube d'insertion (12) avec un piston (16).

2. L'appareil de cicatrisation des blessures (10) de la revendication 1, dans lequel au moins un des membres d'ossature (20) est équipé d'un conduit pour retirer du liquide de la blessure.

3. L'appareil de cicatrisation des blessures (10) de la revendication 2, dans lequel le conduit peut être mis en communication liquide avec un dispositif d'aspiration afin de faciliter le retrait du liquide de la blessure.

4. L'appareil de cicatrisation des blessures (10) de la revendication 2 ou de la revendication 3, dans lequel le conduit peut être adapté pour injecter un médicament dans la blessure.

5. L'appareil de cicatrisation des blessures (10) de n'importe quelle revendication précédente comprenant également au moins un détecteur (72, 74, 76, 78, 80) connecté à au moins un des membres d'ossature (20) ; le ou les détecteurs (72, 74, 76, 78, 80) étant sélectionnés parmi ceux qui suivent : un détecteur de saturation en oxygène, un détecteur de dioxyde de carbone, un détecteur d'électrocardiogramme et un détecteur de pression artérielle.

6. L'appareil de cicatrisation des blessures (10) de la revendication 5, dans lequel le ou les détecteurs comprennent un détecteur de dioxyde de carbone (78) et le tissu bioabsorbable (22) se compose d'un matériau en polyhydroxyalkanoate.

7. L'appareil de cicatrisation des blessures (10) de n'importe quelle revendication précédente comprenant également plusieurs électrodes (72, 74, 76, 78, 80) connectées à au moins un des membres d'ossature, les électrodes (72, 74, 76, 78, 80) pouvant être adaptées pour envoyer une stimulation électrique dans la blessure.

8. L'appareil de cicatrisation des blessures (10) de n'importe quelle revendication précédente, dans lequel les membres d'ossature (20) se composent d'un matériau biodégradable.

9. Un appareil de cicatrisation des blessures (10) conformément à la revendication 1, ledit appareil comportant également un tube d'évacuation et chacun des membres d'ossature (20) comportant un tube flexible raccordé audit tube d'évacuation ; le tissu bioabsorbable (22) étant soutenu par les tubes flexibles (20) ; les tubes flexibles (20) pouvant être adaptés pour retirer du liquide de la blessure à l'aide du tube d'évacuation ; et les tubes flexibles (20) pouvant être retirés de la blessure une fois le tissu bioabsorbable (22) absorbé par la blessure.

10. L'appareil de cicatrisation des blessures (10) de la revendication 9 comprenant un tube d'insertion (12) dans lequel les tubes flexibles (20) et le tissu bioabsorbable (22) sont disposés au départ et un piston (16) opérationnellement raccordé auxdits tubes flexibles (20) ; le tube d'insertion (12) pouvant être inséré dans une blessure et le piston (16) pouvant être utilisé pour déployer les tubes flexibles (20) et le tissu bioabsorbable (22) du tube d'insertion (12) dans la blessure.

11. L'appareil de cicatrisation des blessures (10) de n'importe laquelle des revendications 9 à 10, dans lequel le tube d'évacuation peut être adapté pour être raccordé à un dispositif d'aspiration afin de faciliter le retrait du liquide de la blessure.

12. L'appareil de cicatrisation des blessures (10) de n'importe laquelle des revendications 9 à 11 comprenant également au moins un détecteur connecté à au moins un des tubes flexibles (20) ; le ou les détecteurs étant sélectionnés parmi ceux qui suivent : un détecteur de saturation en oxygène, un détecteur de dioxyde de carbone, un détecteur d'électrocardiogramme et un détecteur de pression artérielle.

13. L'appareil de cicatrisation des blessures (10) de la revendication 12, dans lequel le ou les détecteurs comprennent un détecteur de dioxyde de carbone et le tissu bioabsorbable se compose d'un matériau en polyhydroxyalkanoate.

14. L'appareil de cicatrisation des blessures (10) de n'importe laquelle des revendications 9 à 13, dans lequel le tube d'évacuation et au moins un des tubes flexibles (20) peuvent être adaptés de façon à injecter un médicament dans la blessure.

15. L'appareil de cicatrisation des blessures (10) de n'importe laquelle des revendications 9 à 14, un médicament étant incorporé au tissu bioabsorbable.

16. L'appareil de cicatrisation des blessures (10) de n'importe laquelle des revendications 9 à 15, dans lequel les tubes flexibles (20) se composent d'un matériau biodégradable.

17. L'appareil de cicatrisation des blessures (10) de n'importe laquelle des revendications 9 à 16, comprenant également plusieurs électrodes connectées à au moins un des tubes flexibles ; les électrodes pouvant être adaptées pour envoyer une stimulation électrique dans la blessure.

18. L'appareil de cicatrisation des blessures (10) de n'importe quelle revendication précédente, l'appareil pouvant être adapté pour être placé dans une blessure.
